# FASCICULE DE BREVET EUROPEEN

(11) **EP 2 173 324 B1**
(45) Date de publication et mention de la délivrance du brevet: **10.04.2019**
(21) Numéro de dépôt: 08827889.0
(22) Date de dépôt: 02.07.2008
(51) Int. Cl.: A61K 9/06, A61P 19/00, A61K 31/728, A61K 9/10, A61K 47/10

(54) **GEL INJECTABLE D'ACIDE HYALURONIQUE POUR LE TRAITEMENT DES DEGENERESCENCES ARTICULAIRES**
INJIZIERBARES HYALURONSÄUREGEL ZUR BEHANDLUNG VON GELENKVERSCHLEISS
HYALURONIC ACID INJECTABLE GEL FOR TREATING JOINT DEGENERATION

(30) Priorité: 02.07.2007 FR 0704772
(43) Date de publication de la demande: 14.04.2010
(60) Demande divisionnaire: 19156054.9
(73) Titulaire: Aptissen SA, 1228 Plan-Les-Ouates (CH)
(72) Inventeur: GAVARD MOLLIARD, Samuel, F-74800 La Roche Sur Foron (FR); BENOIT, Olivier, F-74330 Epagny (FR)
(74) Mandataire: Laget, Jean-Loup
(86) Numéro de dépôt international: PCT/FR2008/000948
(87) Numéro de publication internationale: WO 2009/024670

(56) Documents cités:
- EP-A- 0 297 860
- EP-A- 0 499 164
- EP-B1- 1 732 620
- WO-A-2006/067608
- FR-A- 2 865 737
- US-A- 5 095 037
- US-A- 5 106 615
- US-A- 5 972 909
- US-A1- 2003 203 030
- Megacrom, Cromapharma
- Visiol, TRB Chemedica, Ophthalmic Line
- BELDA JOSÉ I ET AL: "Hyaluronic acid combined with mannitol to improve protection against free-radical endothelial damage: experimental model.", JOURNAL OF CATARACT AND REFRACTIVE SURGERY JUN 2005, vol. 31, no. 6, June 2005 (2005-06), pages 1213-1218, XP27831600, ISSN: 0886-3350
- MAZZUCCO DAN ET AL: "Rheology of joint fluid in total knee arthroplasty patients.", JOURNAL OF ORTHOPAEDIC RESEARCH : OFFICIAL PUBLICATION OF THE ORTHOPAEDIC RESEARCH SOCIETY NOV 2002, vol. 20, no. 6, November 2002 (2002-11), pages 1157-1163, XP3028284, ISSN: 0736-0266

## Description

L'invention concerne une formulation aqueuse injectable stérile sous forme de gel composée d'acide hyaluronique (ou l'un de ses sels) avec ou sans autre(s) polysaccharide(s) d'origine naturelle et d'un ou plusieurs polyol(s). Cette formulation injectable est utilisée en intra-articulaire dans le traitement des dégénérescences articulaires.

Une articulation est une jonction permettant de relier deux os et de leur donner une mobilité l'un par rapport à l'autre.

Les articulations synoviales sont les articulations les plus nombreuses, notamment au niveau des membres. Dans ces articulations, les os s'unissent via une cavité remplie d'un liquide à la fois visqueux et élastique appelé liquide synovial.

Le liquide synovial est responsable du bon fonctionnement et de la protection des articulations. Il est notamment constitué d'un polysaccharide, l'acide hyaluronique, qui confère au liquide synovial des propriétés de viscoélasticité permettant, en fonction des contraintes imposées, une lubrification de l'articulation ou une absorption des chocs.

Dans le cas des dégénérescences articulaires comme l'arthrose du genou (dégénérescences dues notamment à des facteurs comme l'obésité, l'hérédité, les traumatismes, ...), le liquide synovial se dégrade (diminution de la concentration et de la masse moléculaire de l'acide hyaluronique) et cette dégradation réduit la capacité du liquide synovial à lubrifier l'articulation et à amortir les chocs.

Le traitement par viscosupplémentation consiste à injecter un gel dans l'articulation afin de remplacer le liquide synovial déficient. La viscosupplémentation peut atténuer ou enrayer la douleur et contribuer à restaurer la mobilité de l'articulation.

Les produits de viscosupplémentation actuellement sur la marché sont des gels qui contiennent de l'acide hyaluronique. Ces gels peuvent être à base d'acide hyaluronique d'origine animale ou non animale et réticulés (cas de Synvisc®, Durolane®) ou non réticulés (cas de Synocrom®, Arthrum®, Lubravisc®, Structovial®).

Il est bien connu de l'homme de l'art que la rémanence d'un gel à base d'acide hyaluronique est faible dans une articulation (de quelques heures à quelques jours). D'après Laurent « The Chemistry, biology and medical applications of hyaluronan and its derivatives, Wenner-Gren International series, Volume 72 », la demi-vie dans une articulation de lapin d'une solution à 1% de hyaluronan est de 12h et celle d'un gel à 0.5% de Hylan B est de 9 jours.

Cette faible rémanence (cinétique de résorption rapide du gel au sein de l'articulation) s'explique par une dégradation (par dépolymérisation) de l'acide hyaluronique. Les principaux facteurs de dégradation de l'acide hyaluronique dans l'articulation sont la dégradation radicalaire, la dégradation thermique à 37°C et la dégradation mécanique (la dégradation enzymatique n'est pas un facteur important de dégradation dans l'articulation). Bien que l'efficacité thérapeutique du viscosupplément soit de plus longue durée que son temps de résidence dans l'articulation, la rémanence d'un gel à base d'acide hyaluronique dans l'articulation est un paramètre prépondérant régissant l'efficacité du produit. Ainsi, plus le gel à base d'acide hyaluronique à un temps de résidence important dans l'articulation et plus le traitement par viscosupplémentation (diminution de la douleur, gain de mobilité) est efficace. Par conséquent, l'augmentation du temps de résidence (rémanence) d'un gel au sein de l'articulation est un point capital pour augmenter l'efficacité d'un traitement par viscosupplémentation à l'aide d'un gel à base d'acide hyaluronique.

Il est bien connu par l'homme de l'art que l'augmentation de la concentration en acide hyaluronique, l'utilisation de hautes masses moléculaires d'acide hyaluronique et les techniques de réticulation / greffage de l'acide hyaluronique permettent d'améliorer la rémanence d'un gel à base d'acide hyaluronique. Toutefois, l'optimisation des différents paramètres listés ci-dessus ne semble pas suffisante pour permettre d'augmenter significativement la rémanence d'un gel à base d'acide hyaluronique en intra-articulaire (la demi-vie, au sein de l'articulation, des gels actuels de viscosupplémentation présents sur le marché n'est au maximum que de quelques jours).

WO 2006/067608 décrit une formulation aqueuse contenant de l'acide hyaluronique et un polyol, dans laquelle le polyol est utilisé comme agent osmogène et dans laquelle le rôle de la stérilisation, s'il y en a une, n'est pas évoqué.

EP 0499 164 décrit des suspensions d'acide hyaluronique dans des polyols différentes qui, une fois implantées dans un organisme vivant, libèrent les polyols en absorbant l'eau des liquides corporels pour former des gels.

EP 0 297 860 décrit un mélange contenant de l'acide hyaluronique et du glycérol permettant la préparation d'un gel mais ne décrit pas de stérilisation. En outre le produit est d'application topique.

US 5095 037 décrit une solution / suspension d' hyaluronate de sodium et de xylitol / glucose mais ne précise pas si elle est sous forme de gel et a été ou non stérilisée.

US 5 972 909 décrit des solutions de lavage d'articulation contenant de l'acide hyaluronique et du glucose qui ne sont pas spécifiquement destinées à être laissées dans l'articulation bien que leur composition permette d'y laisser sans risques des quantités résiduelles.

US 2003/02 03 030 décrit des compositions formant des hydrogels après administration dans un tissu vivant, donc ne subissant pas la stérilisation requise pour obtenir la propriété rhéologique selon la présente demande.

FR 2 865 737 décrit la préparation d'un gel réticulé spécifique ne contenant pas de polyol.

De façon tout a fait inattendue et surprenante, on a mis en évidence :
- que la présence d'un polyol dans une formulation aqueuse stérile à base d'acide hyaluronique permet d'augmenter significativement la résistance aux dégradations de ce gel
- une forte affinité de l'acide hyaluronique et du polyol au sein du gel stérile impliquant une cinétique lente de relarguage du polyol hors du gel : cette affinité entre l'acide hyaluronique et le polyol implique une protection efficace sur le long terme du gel par le polyol par un effet synergique
- pour une composition particulière d'une formulation aqueuse d'acide hyaluronique et d'un polyol, la stérilisation donne à ce gel des propriétés viscoélastiques tout à fait étonnantes en ce qu'elles reproduisent pratiquement les propriétés viscoélastiques du liquide synovial sain - ces propriétés rhéologiques particulières du gel sont maintenues plus longtemps au cours du temps grâce à la protection contre les dégradations induite par la synergie « acide hyaluronique / polyol ».

La présente invention consiste donc en une formulation aqueuse injectable. Cette formulation, utilisée dans le traitement des dégénérescences articulaires, présente dans certains cas (voir exemples 1 et 3)) une rhéologie proche de celle du liquide synovial et toujours une résistance accrue à la dégradation.

L'exemple 4 montre la meilleure résistance d'un gel à base d'acide hyaluronique et d'un polyol lorsque celui-ci est soumis à un test de dégradation radicalaire, thermique et mécanique. Cette meilleure résistance du gel à la dégradation permet une plus longue rémanence du gel injecté en intra-articulaire.

L'exemple 5 montre la meilleure résistance d'un gel à base d'acide hyaluronique et d'un polyol (non couvert par la revendication 1) à la dégradation thermique. Cette meilleure résistance du gel à la dégradation thermique permet une plus longue rémanence du gel injecté en intra-articulaire et une meilleure stabilité de la formulation lors du stockage du produit avant utilisation (point important pour la durée de péremption du produit).

L'exemple 8 démontre la forte affinité entre l'acide hyaluronique et un polyol. Injecté dans l'articulation, la forte affinité entre l'acide hyaluronique et le polyol permet une meilleure résistance du gel à la dégradation sur le long terme par un effet synergique. En effet, dans le cas d'une injection d'une solution de polyol dans l'articulation, le lavage naturel va rapidement éliminer la molécule (= polyol) de l'articulation. Dans le cas d'un gel à base d'acide hyaluronique avec polyol, la forte affinité entre l'acide hyaluronique et le polyol va empêcher le relarguage rapide du polyol hors du gel (et donc son élimination rapide hors de l'articulation) et permettre ainsi une protection efficace sur le long terme du gel par le polyol contre les dégradations.

Les exemples 1 et 3 montrent une rhéologie d'un gel à base d'acide hyaluronique et d'un polyol proche de celle du liquide synovial.

La publication MAZZUCCO D. et al., « Rheology of joint fluid in total knee arthroplasty patients » ; Journal of Orthopaedic Research, 1157-1163, 2002, indique que la fréquence de croisement entre le module élastique G' et le module visqueux G" est égal à 0,41 ± 0,12 Hz pour un liquide synovial sain (non arthosé) du genou. La valeur de cette fréquence de croisement est confirmée par la publication de Fam et al., « Rheological properties of synovial fluids », Biorheology, 44, 59-74, 2007. Dans cette publication, une figure présente la fréquence de croisement entre les modules G' et G" comprise entre 0 et 10 Hz pour un liquide synovial appartenant à une personne jeune ou âgée ou encore pour un liquide synovial arthrosé.
- En dessous de 0,41 Hz : G" > G', le liquide synovial est à dominante visqueuse impliquant une forte lubrification de l'articulation lorsque le patient est au repos.
- En dessus de 0,41 Hz : G' > G", le liquide synovial est à dominante élastique impliquant une forte absorption des chocs lorsque le patient court ou saute.

Selon un aspect de la présente invention, le gel constitué d'une solution aqueuse d'acide hyaluronique (ou l'un de ses sels) avec ou sans autre(s) polysaccharide(s) d'origine naturelle et d'un ou plusieurs polyol(s) possède, après stérilisation, une fréquence f_{c} de croisement entre le module élastique G' et le module visqueux G" proche de 0,41 Hz. Ainsi, le gel possède des propriétés viscoélastiques proches de celle du liquide synovial.

De ce fait, selon un aspect de la présente invention :
- Au dessous de f_{c} : G" > G', le gel est à dominante visqueuse avec une lubrification efficace de l'articulation au repos,
- Au dessus de f_{c} : G' > G", le gel est à dominante élastique avec une absorption efficace des chocs lorsque le patient court ou saute (protection de l'articulation).
Selon un aspect de la présente invention, la fréquence de croisement est comprise entre 0 et 10Hz, de préférence 0.41 ± 0.41 Hz. Une telle rhéologie est donc appropriée aux contraintes mécaniques des articulations et notamment du genou, de la hanche ou des petites articulations. Par conséquent, elle présente un grand intérêt dans le traitement de l'arthrose par viscosupplémentation du genou ou des autres articulations.

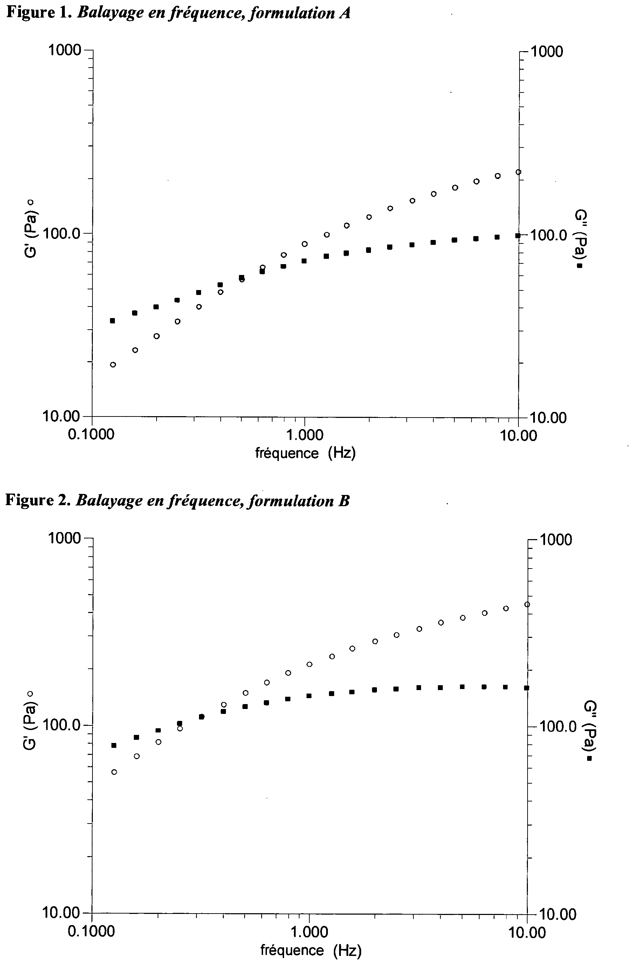

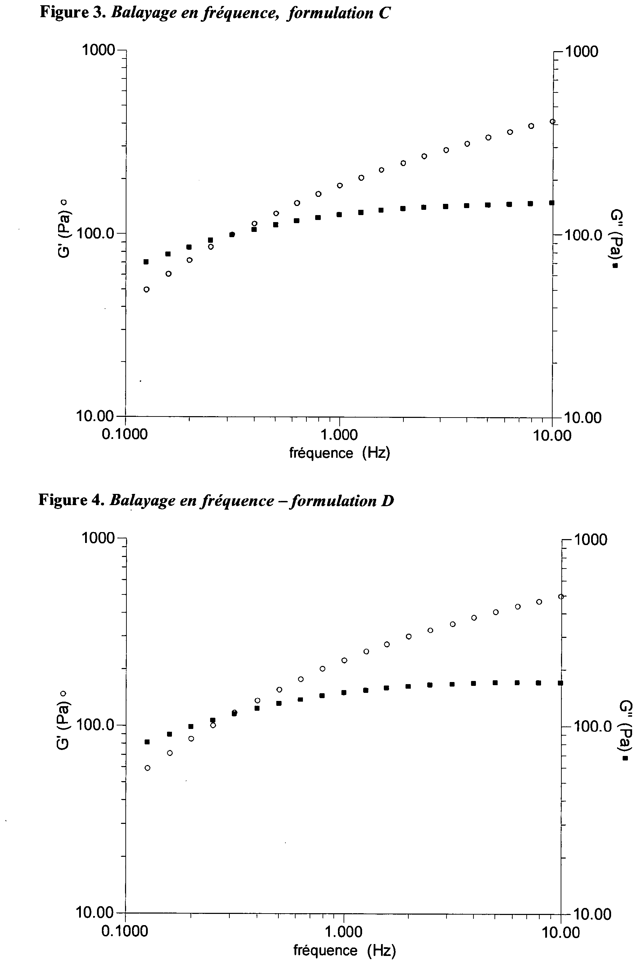

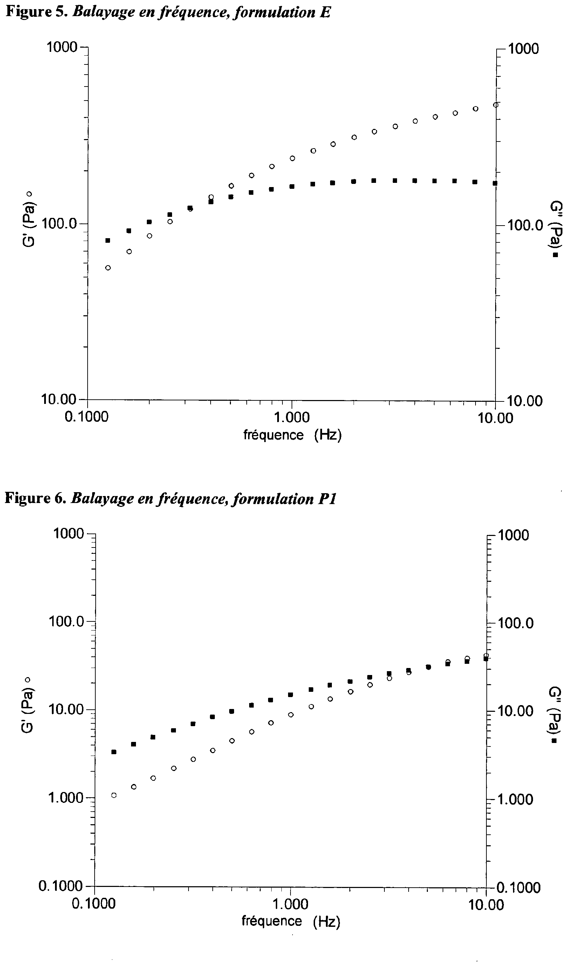

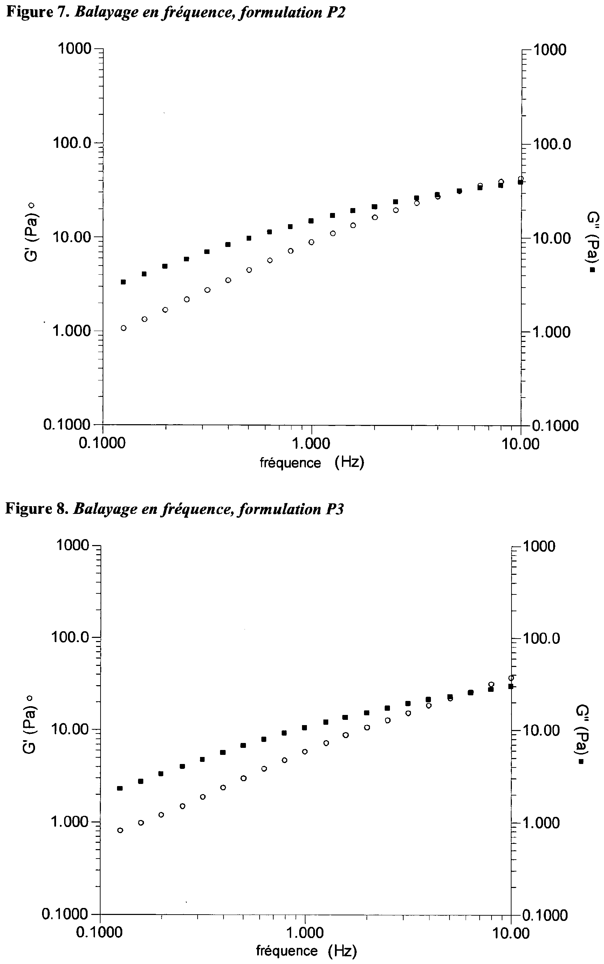

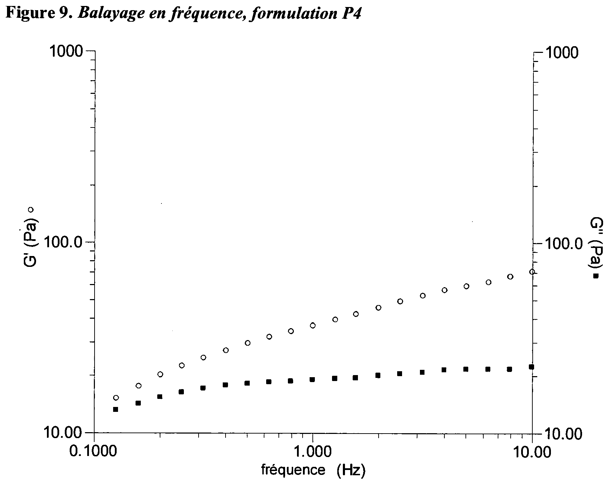

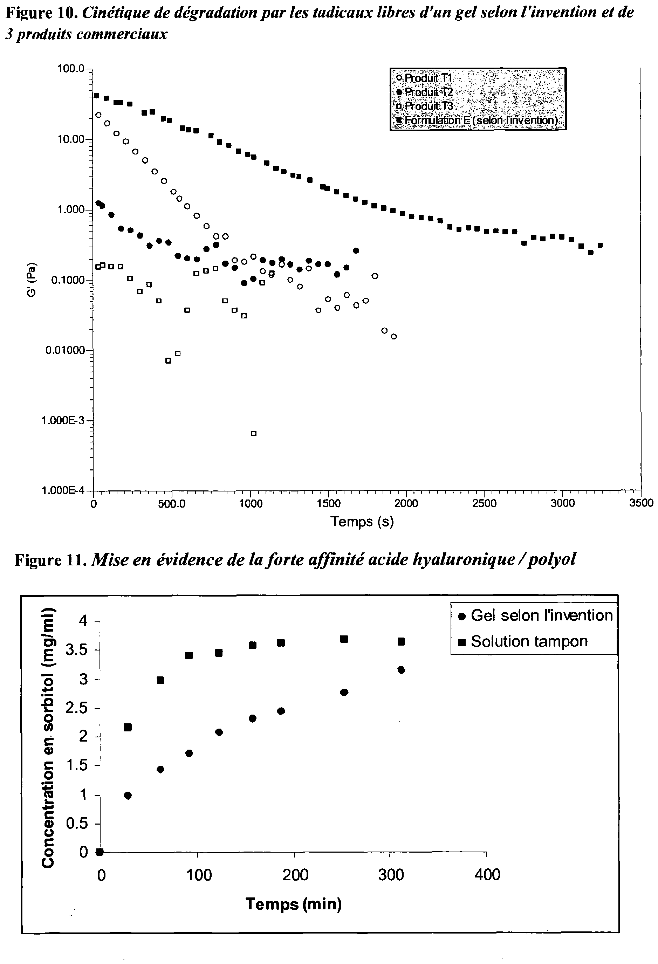

Les figures 3-5 montrent les modules visqueux et élastique de gels selon un aspect de la présente invention tandis que les figures 1 et 2 montrent ceux de gels non couverts par les revendications et les figures 6-9 montrent ceux de gels du commerce. Les figures 10 et 11 illustrent la cinétique de dégradation radicalaire, thermique et mécanique de gels et l'affinité acide hyaluronique / polyol.

L'invention concerne donc l'utilisation d'un gel aqueux injectable stérile tel que revendiqué dans la revendication 1.

L'acide hyaluronique est préférentiellement obtenu par biofermentation mais il peut également être d'origine animale. Sa masse moléculaire est de 0,1 à 10×10⁶Da et de préférence de 2 à 3×10⁶Da.

La concentration en acide hyaluronique est comprise entre 1 et 100 mg/ml et préférentiellement entre 10 et 25 mg/ml.

Le (ou les) polysaccharide d'origine naturelle pouvant être utilisé en association avec l'acide hyaluronique est choisi par exemple parmi la chondroïtine sulfate, le kératane, le kératane sulfate, l'héparine, l'héparane sulfate, la cellulose et ses dérivés, le chitosan, les xanthanes, les alginates, et l'ensemble de leurs sels respectifs.

L'acide hyaluronique, tout comme le ou les polysaccharide(s) d'origine naturelle, peut être réticulé ou non réticulé, greffé ou non greffé selon les techniques de réticulation / greffage décrites dans l'art antérieur.

Le (ou les) polyol non couvert(s) par les revendications pourrai(en)t être choisi par exemple parmi le glycérol, l'érythritol, le xylitol, le lactitol, le maltitol ou encore les oses cycliques tels que le glucose.

La concentration en polyol est comprise entre 0,0001 et 100 mg/ml et préférentiellement entre 15 et 45 mg/ml.

La solution aqueuse utilisée est préférentiellement une solution tamponnée. La composition de cette solution tampon est choisie afin d'avoir les propriétés physico-chimiques (pH, osmolarité) et rhéologiques désirées. Préférentiellement, la solution tampon choisie est une solution tampon phosphate.

Selon la présente invention, la formulation est stérilisée par les techniques bien connues par l'homme de l'art et préférentiellement, à l'autoclave.

La formulation selon la présente invention est utilisée par injection dans l'articulation et la dose injectée peut être comprise entre 0,1 et 20 ml en fonction de la nature de l'articulation traitée.

A titre illustratif, on donne ci-dessous 2 formulations de gels viscoélastiques que l'on peut préparer :
- Gel viscoélastique à base d'acide hyaluronique et de glycérol (non-couvert par les revendications)

Solution stérile composée de 20 mg/ml d'acide hyaluronique (MM = 2,5×10⁶ Da) et de 20 mg/ml de glycérol dans du tampon phosphate.
- Gel viscoélastique à base d'acide hyaluronique et de sorbitol

Solution stérile composée de 20 mg/ml d'acide hyaluronique (MM = 2,5×10⁶ Da) et de 40 mg/ml de sorbitol dans du tampon phosphate.

### Exemples :

Des exemples sont proposés afin d'illustrer l'invention mais me sont nullement limitatifs de ladite invention. Les formulations préparées dans les exemples suivants sont des gels à base de hyaluronate de sodium (NaHA) non réticulé ou réticulé avec polyol.

La préparation des gels non réticulé ou réticulé est effectuée selon les techniques bien connues par l'homme de l'art. Le hyaluronate de sodium utilisé pour fabriquer ces gels possède une masse moléculaire égale à 2.5×10⁶ Da. Dans le cas des gels réticulés, le réticulant utilisé est le BDDE et la définition du taux de réticulation utilisé est : masse(BDDE) / masse(NaHA sec).

L'incorporation du polyol dans le gel est effectuée en ajoutant la quantité nécessaire de polyol dans le gel non réticulé ou réticulé et en mélangeant à la spatule pendant 10 minutes (pour 100 g de gel final).

Les gels préparés sont remplis dans des seringues en verre puis stérilisés à la chaleur humide (T = 121°C).

Le rhéomètre utilisé pour effectuer les mesures rhéologiques est un AR1000 (TA instruments) avec une géométrie plate de 40 mm, un gap de 1000 microns et une température d'analyse de 37°C.

Le dosage des polyols est effectué par une HPLC Ultimate 3000 (Dionex) et une colonne échangeuse d'ions.

### Exemple 1 : Préparation de formulations injectables stériles

Les formulations A et B ne sont pas couvertes par les revendications.
Formulation A : gel à base de NaHA non réticulé avec glycérol
   - 15 mg de NaHA à 2,5×10⁶ Da
   - 20 mg de glycérol
   - Qsp 1 ml de tampon phosphate
Formulation B : gel à base de NaHA non réticulé avec glycérol
   - 20 mg de NaHA à 2,5×10⁶ Da
   - 20 mg de glycérol
   - Qsp 1 ml de tampon phosphate

Les formulations C, D, E et F sont couvertes par les revendications.
Formulation C : gel à base de NaHA non réticulé avec propylène glycol
   - 20 mg de NaHA à 2,5×10⁶ D
   - 15 mg de propylène glycol
   - Qsp 1 ml de tampon phosphate
Formulation D : gel à base de NaHA non réticulé avec mannitol
   - 20 mg de NaHA à 2,5×10⁶ Da
   - 15 mg de mannitol
   - Qsp 1 ml de tampon phosphate
Formulation E : gel à base de NaHA non réticulé avec sorbitol
   - 20 mg de NaHA à 2,5×10⁶ Da
   - 40 mg de sorbitol
   - Qsp 1 ml de tampon phosphate
Formulation F : gel à base de NaHA réticulé avec sorbitol
   - 18 mg de NaHA à 2,5×10⁶ Da, taux de réticulation = 6%
   - 50 mg de sorbitol
   - Qsp 1 ml de tampon phosphate

### Exemple 2 : Propriétés physico-chimiques des formulations de l'exemple 1

- pH (à température ambiante)

| **Formulation** | **pH** |
|---|---|
| A (non couverte) | 7,0 |
| B (non couverte) | 7,2 |
| C | 7,1 |
| D | 7,0 |
| E | 7,1 |
| F | 7,1 |

- osmolarité

| **Formulation** | **Osmolarité (mOsm/kg)** |
|---|---|
| A (non couverte) | 335 |
| B (non couverte) | 322 |
| C | 324 |
| D | 315 |
| E | 326 |
| F | 338 |

*

Les formulations A, B, C, D, E et F sont isotoniques et possèdent un pH neutre.

### Exemple 3 : Propriétés rhéologiques des formulations de l'exemple 1

Les propriétés viscoélastiques des formulations A, B, C, D et E sont caractérisées en mesurant l'évolution du module visqueux (G") et du module élastique (G') en fonction de la fréquence (voir figures 1 à 5).

Pour ces 5 formulations, on constate que la fréquence de croisement du module G' et du module G" est proche de celle du liquide synovial sain.

Le tableau ci-dessous donne les valeurs de fréquence de croisement f_{c} pour chaque formulation et pour le liquide synovial sain.

| **Formulation** | **Fréquence de croisement f_{c} (Hz)** |
|---|---|
| A (non couverte) | 0,50 |
| B (non couverte) | 0,32 |
| C | 0,32 |
| D | 0,32 |
| E | 0,33 |
| Liquide synovial sain (*publication de Mazzucco D. et al*.) | 0,41 ± 0,12 |

Comme décrit dans la présente invention :
- Au dessous de f_{c} : G" > G', le gel est à dominante visqueuse avec une lubrification efficace de l'articulation au repos,
- Au dessus de f_{c} : G' > G", le gel est à dominante élastique avec une absorption efficace des chocs lorsque le patient court ou saute

### Exemple 4 : Résistance aux dégradations des formulations de l'exemple 1

Pour montrer que la présence d'un polyol dans un gel à base de NaHA permet de réduire la dégradation du gel par une action radicalaire, thermique et mécanique, on a comparé la résistance à la dégradation de gels à base de NaHA avec polyol (formulations de l'exemple 1) et la résistance à la dégradation de gels à base de NaHA sans polyol (= gels références).

Pour les formulations B, C, D et E de l'exemple 1, le gel référence à base de NaHA sans polyol est un gel à base de NaHA non réticulé à 20 mg/ml de NaHA (MM= 2,5×10⁶ D, dans du tampon phosphate) - formulation G.

Pour la formulation F de l'exemple 1, le gel référence à base de NaHA sans polyol est un gel à base de NaHA réticulé à 18 mg/ml de NaHA (MM= 2,5×10⁶ D avant réticulation, dans du tampon phosphate) possédant un taux de réticulation de 6% - formulation H.

Le test de dégradation est effectué en ajoutant un oxydant dans le gel à tester, en homogénéisant à la spatule pendant 1 minute, en se plaçant à la température de 37°C et en imposant une déformation de 0.3%. La valeur du paramètre tanδ=G"/G' à 0,7Hz (paramètre caractéristique des propriétés viscoélastiques du gel) est mesurée au cours du temps.

On constate que ce paramètre augmente au cours du temps, synonyme d'une déstructuration progressive du gel. Les valeurs mesurées à t=0 et t=15 min pour les formulations B, C, D, E, F, G et H sont données dans le tableau ci-dessous.

| **Formulation** | **Tanδ (t = 0 min)** | **Tanδ (t = 15 min)** | **Δ Tanδ (%)** |
|---|---|---|---|
| B (non couverte) | 1,10 | 3,34 | + 204 % |
| C | 1,08 | 3,13 | + 205 % |
| D | 1,19 | 4,63 | + 289% |
| E | 1,08 | 2,57 | + 138% |
| F | 0,74 | 0,80 | +8% |
| G | 1,41 | 6,56 | + 365 % |
| H (réf.) | 0,74 | 0,93 | +26% |

Comme décrit dans la présente invention, chaque formulation B, C, D et E possède une résistance aux dégradations significativement plus importante que celle du gel sans polyol (formulations G). De même, la formulation F possède une résistance aux dégradations significativement plus importante que celle du gel correspondant sans polyol (formulations H).

Par conséquent, les polyols protègent le gel de façon efficace contre les dégradations.

### Exemple 5 : Etude de vieillissement accéléré d'une formulation avec et sans polyol

Deux formulations sont mises en vieillissement accéléré à l'étuve à 40°C :
- formulation B (non couverte par les revendications) de l'exemple 1: solution à base d'acide hyaluronique et de glycérol
- formulation G sans adjonction d'alcool (décrite dans l'exemple 4)
   - 20 mg d'acide hyaluronique à 2,5×10⁶ Da
   - Qsp 1 ml de tampon phosphate

Une mesure de la viscosité zéro (viscosité à cisaillement nul) et une détermination de la fréquence f_{c} de croisement entre le module élastique G' et le module visqueux G" est effectuée à 3 temps (t = 0, 7 jours, 26 jours)

Les résultats obtenus sont donnés dans le tableau ci-dessous :

| **Nombre de jours de vieillissement à 40°C** | **Formulation** | **Viscosité zéro** | **Variation de viscosité zéro par rapport à to (%)** | **f_{c} (Hz)** | **Variation de fc par rapport à to (%)** |
|---|---|---|---|---|---|
| O jour | B | 252 | / | 0.32 | / |
| | G | 192 | / | 0,39 | / |
| 7 jours | B | *ND | / | 0,32 | 0 % |
| | G | *ND | / | 0,39 | 0 % |
| 26 jours | B | 210 | -17 % | 0,37 | + 16 % |
| | G | 143 | -26 % | 0,50 | + 28 % |

| | | | | | |
|---|---|---|---|---|---|
| *ND : non déterminé | | | | | |

On constate que lors du vieillissement accéléré, la perte de viscosité zéro et le décalage de la fréquence de croisement f_{c} sont moins importants dans le cas de la formulation B (formulation selon la présente invention) que dans le cas de la formulation sans polyol (formulation G).

### Exemple 6 : Comparaison de la rhéologie de 4 produits commerciaux de viscosupplémentation et d'une formulation obtenue selon la présente invention

Les produits testés sont les suivants :

| **Produit** | **Nom commercial** | **Fabricant** | **Concentration en acide hyaluronique (mg/ml)** | **Masse moléculaire de l'acide hyaluronique (Da)** | **Méthode de stérilisation** |
|---|---|---|---|---|---|
| P1 | Synocrom® | CROMA PHARMA | 10 | 2,2 - 2,7.10⁶ | chaleur humide |
| P2 | Structovial® | CROMA PHARMA | 10 | 2,2 - 2,7.10⁶ | chaleur humide |
| P3 | Fermathron® | HYALTECH | 10 | 1.10⁶ | filtration |
| P4 | Lubravisc® | BOHUS BIOTECH | 10 | 4.10⁶ | chaleur humide |
| Formulation D de l'exemple 1 | Non applicable | Non applicable | 20 (+ 15 mg/ml de mannitol) | 2.5.10⁶ | chaleur humide |

Pour les 5 gels testés, les figures 6 - 9 donnent les modules visqueux (G") et élastique (G') en fonction de la fréquence.

On constate que seul le gel selon l'exemple 1 présente une fréquence de croisement (0,32 Hz) voisine de celle du liquide synovial sain (0,41 Hz).

Le tableau ci-dessous regroupe les valeurs des fréquences de croisement f_{c} pour les produits P1 à P4 et pour la formulation A de l'exemple 1.

| **Produit** | **fc (Hz)** |
|---|---|
| Formulation D de l'exemple 1 | 0,32 |
| P1 | 5,8 |
| P2 | 5,0 |
| P3 | 6,3 |
| P4 | 0,09 |

On sait d'après la publication de Mazzucco D. et al. (citée plus haut) que la fréquence de croisement du liquide synovial sain (0,41 Hz) est en dessous des fréquences observées dans le genou lors de la marche (0,7 Hz) et de la course (3 Hz).

Pour les produits P1 à P3, la fréquence de croisement est bien supérieure à 3 Hz et par conséquent, les produits ne présentent pas une forte élasticité permettant l'absorption des chocs lorsque le genou est en mouvement.

Le produit P4 a une très faible fréquence de croisement, le module élastique est supérieur au module visqueux sur toute la gamme de fréquence 0,1 - 10 Hz. Par conséquent, l'élasticité est importante lorsque le genou est en mouvement mais la lubrification de l'articulation est peu efficace lorsque le patient est au repos.

### Exemple 7 : Comparaison de la résistance aux dégradations de 3 produits commerciaux de viscosupplémentation et d'une formulation obtenue selon la présente invention

Les produits testés sont les suivants :

| **Produit** | **Nom commercial** | **Fabricant** | **Concentration en acide hyaluronique (mg/ml)** |
|---|---|---|---|
| T1 | Arthrum® | LCA Pharmaceutical | 20 |
| T2 | Ostenil® | TRB Chemedica | 10 |
| T3 | Synocrom® | CROMA PHARMA | 10 |
| Formulation E de l'exemple 1 | Non applicable | Non applicable | 20 (+ 40 mg/ml de sorbitol) |

Le test de dégradation est effectué selon la méthode décrite dans l'exemple 4.

La valeur du paramètre G' à 0,7Hz est suivi au cours du temps.

Les courbes de rhéologie ainsi obtenues sont données en figure 10.

On constate que le gel selon la présente invention se dégrade significativement moins rapidement que les 3 produits commerciaux testés.

### Exemple 8 : Mise en évidence de la forte affinité acide hyaluronique / polyol

Afin de démontrer la forte affinité entre l'acide hyaluronique et le polyol et donc la protection du gel par le polyol sur le long terme, une étude de suivi du relarguage d'un polyol par dialyse a été effectuée.

5g de la formulation E (gel à base de 20 mg/ml de NaHA non réticulé et de 40 mg/ml de sorbitol - exemple 1) a été introduit dans une membrane (n°1) de dialyse (Spectra/Pore®, MWCO : 12-14,000).

5g d'une solution tampon phosphate contenant 40 mg/ml de sorbitol a été introduit dans une 2^{ème} membrane (n°2) de dialyse (Spectra/Pore®, MWCO : 12-14,000) - même dimension que la membrane n°1.

Ces membranes ont été disposées dans des flacons respectifs contenant 50 g d'eau purifiée (= bain de dialyse) sous agitation magnétique. Des mesures de concentration en sorbitol par HPLC ont été effectuées dans les bains de dialyse à différents temps afin de suivre la cinétique de relarguage du sorbitol hors de la membrane avec gel ou solution tampon.

Les courbes de suivi de la concentration en sorbitol au cours du temps sont données en figure 11.

La cinétique de relarguage du sorbitol dans un gel est significativement plus lente que dans une solution tampon.

Cette étude met en avant la synergie entre l'acide hyaluronique et le polyol présent dans le gel : la forte affinité acide hyaluronique / polyol permet au polyol d'être présent au sein du gel sur une longue période et la capacité de protection du polyol vis-à-vis du gel permet d'avoir une forte résistance sur le long terme du gel contre les dégradations.

## Revendications

1. Utilisation d'un gel aqueux injectable stérile de polysaccharide(s) d'origine naturelle contenant de l'acide hyaluronique ou un de ses sels à une teneur de 1 à 100 mg/ml et éventuellement un ou plusieurs autres polysaccharides d'origine naturelle, et un ou plusieurs polyols choisi(s) parmi le propylène glycol, le sorbitol, le mannitol seul ou en mélange, à une teneur de 0,0001 à 100 mg/ml, après stérilisation à la chaleur effectuée à l'autoclave du mélange de polysaccharide(s) et de polyol(s), le gel obtenu possédant une fréquence de croisement du module élastique G' et du module visqueux G" comprise entre 0 et 10 Hz, de préférence entre 0.41 ± 0.41 Hz, G' étant supérieur à G" à haute fréquence, pour la préparation d'une formulation injectable utilisée dans le traitement des dégénérescences articulaires par injection intra-articulaire.

2. Utilisation selon la revendication 1, dans laquelle l'acide hyaluronique est non réticulé ou sensiblement non réticulé.

3. Utilisation selon la revendication 1, dans laquelle l'acide hyaluronique est réticulé.

4. Utilisation selon l'une quelconque des revendications 1 à 3, dans laquelle l'acide hyaluronique ou un de ses sels est présent à une teneur de 10 à 25 mg/ml et le ou les polyols sont présents à une teneur de 15 à 45 mg/ml.

5. Utilisation selon l'une quelconque des revendications 1 à 4, dans laquelle l'acide hyaluronique (ou un de ses sels) seul ou en mélange à une masse moléculaire de 0,1 à 10x10⁶ Da.

6. Utilisation selon l'une quelconque des revendications 1 à 5, dans laquelle l'acide hyaluronique a une masse moléculaire de 2 à 3x10⁶ Da.

7. Utilisation selon l'une quelconque des revendications 1 à 6, dans laquelle l'acide hyaluronique est présent à une teneur de 20 mg/ml et du sorbitol est présent à une teneur de 40 mg/ml.

8. Utilisation selon l'une quelconque des revendications 1 à 7, dans laquelle la formulation est injectée à raison de 0,1 à 20 ml.

## Patentansprüche

1. Verwendung eines natürlichen sterilen injizierbaren wässrigen Polysaccharidgels, das Hyaluronsäure oder eines ihrer Salze in einem Gehalt von 1 bis 100 mg/ml und eventuell ein oder mehrere andere natürliche Polysaccharide und ein oder mehrere Polyole, ausgewählt aus dem Propylenglycol, dem Sorbitol, dem Mannitol allein oder gemischt, in einem Gehalt von 0,0001 bis 100 mg/ml enthält, nach Sterilisation durch Wärme, durchgeführt im Autoklav, des Gemischs aus Polysaccharid(en) und aus Polyol(en), wobei das erhaltene Gel eine Kreuzfrequenz des elastischen Moduls G' und des viskosen Moduls G" besitzt, das zwischen 0 und 10 Hz, vorzugsweise 0,41 ± 0,41 Hz, liegt, wobei G' größer ist als G" bei hoher Frequenz, für die Herstellung einer injizierbaren Formulierung, die bei der Behandlung von Gelenkdegeneration durch intraartikuläre Injektion verwendet wird.

2. Verwendung nach Anspruch 1, wobei die Hyaluronsäure nicht vernetzt oder im Wesentlichen nicht vernetzt ist.

3. Verwendung nach Anspruch 1, wobei die Hyaluronsäure vernetzt ist.

4. Verwendung nach einem der Ansprüche 1 bis 3, wobei die Hyaluronsäure oder eines ihrer Salze in einem Gehalt von 10 bis 25 mg/ml vorhanden sind und das oder die Polyole in einem Gehalt von 15 bis 45 mg/ml vorhanden sind.

5. Verwendung nach einem der Ansprüche 1 bis 4, wobei die Hyaluronsäure (oder eines ihrer Salze) allein oder gemischt eine molekulare Masse von 0,1 bis 10x10⁶ Da hat.

6. Verwendung nach einem der Ansprüche 1 bis 5, wobei die Hyaluronsäure eine molekulare Masse von 2 bis 3x10⁶ Da hat.

7. Verwendung nach einem der Ansprüche 1 bis 6, wobei die Hyaluronsäure in einem Gehalt von 20 mg/ml vorhanden ist und Sorbitol in einem Gehalt von 40 mg/ml vorhanden ist.

8. Verwendung nach einem der Ansprüche 1 bis 7, wobei die Formulierung in einer Menge von 0,1 bis 20 ml injiziert wird.

## Claims

1. Use of a sterile, injectable aqueous gel of polysaccharide(s) of natural origin containing hyaluronic acid or one of the salts thereof in a content of 1 to 100 mg/ml and optionally one or more other polysaccharides of natural origin, and one or more polyols selected from among propylene glycol, sorbitol, mannitol alone or in a mixture in a content of 0.0001 to 100 mg/ml, after heat sterilisation conducted in an autoclave of the mixture of polysaccharide(s) and polyol(s), the gel obtained having a crossover frequency of the elastic modulus G' and viscous modulus G" of between 0 and 10 Hz, preferably between 0.41 ± 0.41 Hz, G' being higher than G" at high frequency, for preparing an injectable formulation used in the treatment of joint degeneration via intra-articular injection.

2. The use according to claim 1, wherein the hyaluronic acid is non-crosslinked or substantially non-crosslinked.

3. The use according to claim 1, wherein the hyaluronic acid is crosslinked.

4. The use according to any of claims 1 to 3, wherein the hyaluronic acid or one of the salts thereof is contained in a content of 10 to 25 mg/ml and the polyol(s) are contained in a content of 15 to 45 mg/ml.

5. The use according to any of claims 1 to 4, wherein the hyaluronic acid (or one of the salts thereof) alone or in a mixture has a molecular weight of 0.1 to 10x10⁶ Da.

6. The use according to any of claims 1 to 5, wherein the hyaluronic acid has a molecular weight of 2 to 3x10⁶ Da.

7. The use according to any of claims 1 to 6, wherein the hyaluronic acid is contained in a content of 20 mg/ml and sorbitol is contained in a content of 40 mg/ml.

8. The use according to any of claims 1 to 7, wherein the formulation is injected in a proportion of 0.1 to 20 ml.
